**Europäisches Patentamt**

**European Patent Office**

**Office européen des brevets**

(19)

(11) Veröffentlichungsnummer : **0 038 900**
**B1**

(12) # EUROPÄISCHE PATENTSCHRIFT

(45) Veröffentlichungstag der Patentschrift :
21.03.84

(51) Int. Cl.³ : **C 01 B 33/12, C 08 K 3/36**

(21) Anmeldenummer : **81100412.6**

(22) Anmeldetag : **21.01.81**

(54) Verfahren zur Herstellung pyrogener Kieselsäure.

(30) Priorität : 25.04.80 DE 3016010

(43) Veröffentlichungstag der Anmeldung :
04.11.81 Patentblatt 81/44

(45) Bekanntmachung des Hinweises auf die Patenterteilung : 21.03.84 Patentblatt 84/12

(84) Benannte Vertragsstaaten :
**AT BE CH DE FR GB IT LI LU NL SE**

(56) Entgegenhaltungen :
**DE-A- 2 107 082**
**DE-A- 2 403 783**
**DE-A- 2 620 737**
**DE-A- 2 904 199**
**DE-B- 1 933 291**
**DE-B- 2 123 233**
**DE-B- 2 344 388**
**US-A- 3 086 851**
**US-A- 3 772 427**

(73) Patentinhaber : **Degussa Aktiengesellschaft**
**Weissfrauenstrasse 9**
**D-6000 Frankfurt am Main 1 (DE)**

(72) Erfinder : **Schwarz, Rudolf, Dr.**
**Taunusstrasse 2**
**D-8755 Alzenau (DE)**
Erfinder : **Kleinschmit, Peter, Dr.**
**Wildaustrasse 19**
**D-6450 Hanau 9 (DE)**

# 0 038 900

## Verfahren zur Herstellung pyrogener Kieselsäure

Die Herstellung pyrogener hochoberflächiger Kieselsäuren durch Hochtemperaturhydrolyse von Siliciumhalogeniden, insbesondere von Siliciumtetrachlorid in einer Wasserstoffflamme, ist nicht neu. Seit Jahrzehnten werden nach diesem Verfahren große Mengen sehr reiner hochdisperser Kieselsäuren hergestellt, die als Verdickungsmittel in der Lack- und Farbenchemie, bei der Herstellung pasteuser oder salbenartiger Massen, z. B. bei der Zahnpastenbereitung und in der pharmazeutischen Industrie eine sehr große Verbreitung gefunden haben.

Es ist bekannt, daß der Grad der mit der pyrogenen Kieselsäure erzielbaren Verdickung von vielerlei Faktoren abhängig ist. Insbesondere bestehen Abhängigkeiten zur Art des Systems, das verdickt werden soll, zur Kieselsäuretype und ihrer angewendeten Konzentration, zum pH-Wert des Systems und zum Grad der Dispergierung, der mit einem bestimmten Dispergierungsaufwand erreicht wurde.

Dem Einfluß der Kieselsäuretype kommt eine besonders große Bedeutung zu. Die Verdickung eines Systems ist nämlich um so größer, je höher die Aktivität der verwendeten Kieselsäuretype ist. Als Maß für die Aktivität kann man vereinfachend die äußere spezifische Oberfläche einer Kieselsäure ansehen. Aus diesem Grund sind auch die pyrogenen Kieselsäuretypen nach der Größe der spezifischen Oberfläche geordnet. Es trifft aber nicht zu, daß bei einem gegebenen Dispergierungsaufwand die Kieselsäuretype mit der höchsten spezifischen Oberfläche auch die größte Verdickungswirkung ausübt. Vielmehr ist es im allgemeinen so, daß die Verdickungswirkung mit steigender spezifischer Oberfläche der Kieselsäure zunächst bis zu einem Maximum zunimmt und sich bei weiter steigender Oberfläche wieder vermindert. So hat zum Beispiel eine handelsübliche pyrogene Kieselsäure in einem ungesättigten Polyester-Testharz bei gegebener Konzentration folgende Verdickungswirkungen in Abhängigkeit von der spezifischen Oberfläche :

| Spez. Oberfläche $m^2/g$ | Viskosität mPas |
|---|---|
| 130 | 2.000 |
| 200 | 2.900 |
| 300 | 3.500 |
| 380 | 3.000 |

Wie man sieht, kommt man bei einer gegebenen Konzentration an Kieselsäure über eine Viskosität von ca. 3.500 mPas nicht hinaus.

Es gibt zwar zahlreiche betriebstechnische Möglichkeiten bei der Herstellung pyrogener Kieselsäuren zu noch höheren spezifischen Oberflächen zu gelangen. Sie alle führen aber, wie gezeigt wurde, bei gleichen mengenmäßigen Verhältnissen nicht zu einer Verbesserung der verdickenden Wirkung der pyrogenen Kieselsäure. Andererseits sind die betriebstechnischen Möglichkeiten, die verdickende Wirkung von pyrogener Kieselsäure bei gegebener spezifischer Oberfläche zu verbessern, sehr eng begrenzt oder erfordern einen unvertretbaren hohen Aufwand.

Aus der DE-OS 2 904 199 Beispiel 4 ist eine pyrogen hergestellte Kieselsäure mit einer spezifischen BET-Oberfläche von 309 $m^2/g$ und einer Verdickung von 4 040 mPascal bekannt.

Es wurde nun gefunden, daß sich bei Verwendung von Siloxan oder einem Gemisch von verschiedenen Siloxanen als Rohstoff für die Herstellung von pyrogener Kieselsäure unter bestimmten Betriebsbedingungen Produkte erhalten lassen, die sowohl in organischen Flüssigkeiten als auch in wäßerigen Systemen eine ungewöhnlich hohe verdickende Wirkung ausüben.

Gegenstand der Erfindung ist ein Verfahren zur pyrogenen Herstellung von Kieselsäure mit einer spezifischen BET-Oberfläche von 250 ± 25 bis 350 ± 25 $m^2/g$ und einer Verdickung von 4 000 bis 8 000 mPas in ungesättigten Polyestern, dadurch gekennzeichnet, daß man Siloxan oder ein Gemisch von verschiedenen Siloxanen in Gegenwart von Wasserstoff oder einem Kohlenwasserstoff verbrennt.

Als Siloxan kann man Octamethyltrisiloxan, 1,1,3,3,5,5-Hexamethylcyclotrisiloxan oder 1,1,3,3,5,5,7,7,-Octamethylcyclotetrasiloxan verwenden.

In einer bevorzugten Ausführungsform der Erfindung kann man als Siloxan Hexamethyldisiloxan verwenden.

Ein Vorteil der Verwendung dieses Rohstoffes liegt in seiner Chlorfreiheit, die einen Verzicht auf eine sonst unbedingt erforderliche nachträgliche Entsäuerung der Kieselsäure mit einem Wasserdampf/Luftgemisch bei hoher Temperatur ermöglicht.

Aufgrund des sehr hohen Energieinhaltes von Hexamethyldisiloxan ist es möglich, diese Substanz als Dampf/Luftgemisch in einem geeigneten Brenner ohne Zusatz von z. B. Wasserstoff abzubrennen, wobei Produkte mit Oberflächen in Abhängigkeit von den Betriebseinstellungen erhalten werden können, wie das mit anderen bekannten Rohstoffen, z. B. Siliciumtetrachlorid, möglich ist. Allerdings ist die verdickende Wirkung dieser so hergestellten Produkte nicht so ausgeprägt, wie wenn man zusätzlich Wasserstoff mit in das Dampf/Luftgemisch einbringt und unter Berücksichtigung ausreichender Luftmengen gemeinsam mit dem Hexamethyldisiloxan abbrennen läßt.

2

**0 038 900**

Gemäß der Erfindung muß man daher das Siloxan in Gegenwart von Wasserstoff oder einem Kohlenwasserstoff verbrennen.

Auf diesem Wege konnten Produkte erhalten werden, die eine bisher nicht gekannte verdickende Wirkung auf die verschiedensten flüssigen Systeme ausüben.

Die folgenden Beispiele sollen den Erfindungsgegenstand näher erläutern und beschreiben :

## Vergleichsbeispiel 1

In einem Verdampfer werden 3,4 Liter Siliciumtetrachlorid pro Stunde, das sind 5,03 kg, verdampft und mit 4,3 $m^3$ auf 80 °C erwärmte Luft vermischt. Das Silan/Luftgemisch wird sodann in das Schaftende des Brenners, gemäß US-PS 3 086 851 tangential eingeleitet und dort mit 1,76 $m^3$/h Wasserstoff, der durch einen zweiten Stutzen einströmt, homogen vermischt. Die sternförmigen Einbauten des Brennerschaftes bewirken eine Laminierung der Gasgemische, die an der Brenneröffnung mit einer Geschwindigkeit von 23,8 m/sec austritt und dort nach der Zündung abbrennt. Der Brenneraustritt hat einen Durchmesser von 10 mm. Zur Stabilisierung der Flamme und zur Verhinderung von Ansätzen am Brennerende läßt man durch einen die Brennermündung umgebenden Ringspalt 0,35 $m^3$/h Wasserstoff austreten und als Flammenmantel ebenso abbrennen. Die heißen Reaktionsprodukte der Flammenzone werden in eine Kühlkammer eingesaugt und nach der Abkühlung auf < 150 °C pneumatisch einer Filtrationsanlage zugeführt. In der Filteranlage trennt man die erhaltene pyrogene Kieselsäure von den gasförmigen Reaktionsprodukten Sauerstoff, Stickstoff, Wasserdampf und Chlorwasserstoff. Die noch etwa 1 % Chlor enthaltende Kieselsäure wird anschließend in einer auf 600 °C geheizten Reaktionsstrecke mit Hilfe eines Wasserdampf/Luftgemisches nachentsäuert und schließlich in einem Bunker abgesetzt. Die Ausbeute pro Stunde beträgt 1,78 kg $SiO_2$.

Die spezifische Oberfläche der erhaltenen Kieselsäure bestimmt nach BET beträgt 215 $m^2$/g. Mit diesem Produkt läßt sich ein ungesättigtes Polyesterharz in einem Test, wie er weiter unten beschrieben ist, auf eine Viskosität von 2.980 mPas bringen. Mit dem gleichen Produkt erreicht man in einer Zahnpasta-Testrezeptur, die weiter unten ist, eine Verdickung von 2.200 mPas.

## Vergleichsbeispiel 2

In gleicher Weise wir im Beispiel 1 werden 0,546 Liter Hexamethyldisiloxan ≙ 0,752 kg pro Stunde verdampft und mit 7,11 $m^3$/h auf 120 °C vorerwärmter Luft vermischt. Das Siloxan/Luftgemisch überführt man in den Brenner gemäß US-PS 3 086 851 und läßt es an der Brenneröffnung mit einer Geschwindigkeit von ca. 25,5 m/sec ausströmen und abbrennen. Die Reaktionsflamme ist wie im Beispiel 1 mit einer reiner Wasserstofflamme von 0,35 $m^3$ $H_2$/H eingemantelt. Nach dem Abkühlen der Reaktionsprodukte aus der Flamme in der Kühlzone auf < 150 °C wird die erhaltene Kieselsäure von der gasförmigen Reaktionsmischung aus Stickstoff, Sauerstoff, Wasserdampf und Kohlendioxid durch eine Filtration oder mit Hilfe eines Zyklons abgetrennt.

Es werden pro Stunde 0,55 kg pyrogene Kieselsäure erhalten. Die spezifische Oberfläche nach BET der Kieselsäure beträgt 159 $m^2$/g. Die Viskosität eines nach der Prüfvorschrift gemäß Beispiel 1 verdickten ungesättigten Polyesterharzes liegt bei 1.575 mPas.

## Vergleichsbeispiel 3

Gemäß Beispiel 1 werden 0,819 Liter ≙ 1,13 kg Hexamethyldisiloxan verdampft, mit 16,86 $Nm^3$ auf 120 °C erwärmter Luft vermischt und in einen Brenner der beschriebenen Konstruktion eingeleitet. Gleichzeitig werden 1,46 $m^3$ Wasserstoff in den Brenner eingeleitet und mit den anderen beiden Komponenten homogen vermischt. Die drei Ausgangsstoffe strömen mit einer Geschwindigkeit von 65, 4 m/sec aus und brennen nach der Zündung mit rauschender Flamme ab. Die Mantelwasserstoffmenge beträgt wieder 0,35 $m^3$/h. Eine Nachentsäuerung der nach Abkühlung der Reaktionsprodukte und nach Abtrennung der gasförmigen Stoffe erhaltenen Kieselsäure (0,83 kg $SiO_2$/h), wie im Beispiel 1 ist nicht erforderlich. Die spezifische Oberfläche nach BET der erhaltenen Kieselsäure beträgt 334 $m^2$/g. Die Viskosität des ungesättigten, mit diesem Versuchsprodukt verdickten Polyestertestlackes liegt mit 7.595 mPas ungewöhnlich hoch.

In einer Zahnpasten-Grundmasse erreicht man gemäß dem beschriebenen Test eine Verdickung von 4.300 mPas. Die Kieselsäure läßt sich in der Grundmasse sehr gut dispergieren. Die Homogenität der verdickten Grundmasse ist ebenfalls gut. Es lassen sich keine störenden Gritteilchen feststellen. Bei mehrtägiger Lagerung der verdickten Grundpaste kommt es zu einem weiteren Viskositätsanstieg, der schließlich Werte von mehr als 6.000 mPas erreicht.

## Vergleichsbeispiel 4

Zur Herstellung einer Kieselsäure mit geringerer spezifischer Oberfläche und einem gegenüber dem Ergebnis von Beispiel 3 abgesenkten Verdickungsverhalten wird entsprechend dem Beispiel 3 verfahren, mit dem Unterschied, daß nur 15,08 $m^3$ Luft zur Anwendung kommen. Die Ausströmgeschwindigkeit der 3-Komponentenmischung aus dem Brenner beträgt in diesem Fall 59 m/sec. Die spezifische Oberfläche der erhaltenen Kieselsäure beträgt 241 m²/g und die Viskosität des ungesättigten Polyesterharzes gemäß Beispiel 1 liegt bei 4.405 mPas.

Prüfung des Verdickungsverhalten von pyrogener Kieselsäure bei Dissolverdispergierung

1. Grundlage

Maßstab des Verdickungsverhaltens ist die Viskosität eines mit pyrogener Kieselsäure vermischten Polyesterharzes.

Die Einheit der dynamischen Viskosität ist die Pascalsekunde (Pas). Eine Millipascalsekunde (mPas) entspricht der bisher gebräuchlichen Einheit Centipoise (cP). Die Bestimmung der Viskosität erfolgt mit einem Rotationsviskosimeter nach DIN 53 214.

2. Geräte und Reagenzien

Rotovisko RV 1-3 oder Nachfolgetypen (Firma Haake, Karlsruhe) mit Wasserthermostat, Prüfkörper MV 2 und Meßkopf 50.

Dissolver, ∅ der Dispergierschreibe 5 cm.

Plastikbecher, äußerer Durchmesser 8,4 cm.

Ludopal P6 (BASF, mit einer Viskosität von 1 100 ± 100 mPas).

Monostyrollösung (100 g Monostyrol und 0,4 g Paraffin).

Tafelparaffin 50/52 °C, Ölgehalt 1-1,5 %, Firma Jung, Atlantic Refining GmbH, Hamburg.

3. Durchführung

In 142,5 Gew.-Teile Ludopal$^R$ P6 werden 7,5 Gew.-Teile pyrogene Kieselsäure in einem Plastikbecher eingewogen (mit dem Spatel von Hand vermischt) und mit einem Dissolver fünf Minuten lang bei 3 000 Upm dispergiert (Dissolverscheibe ca. 1 mm von Bodem des Plastikbechers entfernt).

60 g der erhaltenen Paste werden mit 63 g Polyesterharz und 27 g Monostyrollösung versetzt, mit einem Spatel gut verrührt und in dem genannten Plastikbecher mit dem Dissolver drei Minuten bei 1 500 Upm dispergiert.

Um Verluste an Styrol zu vermeiden, wird der Becher während der Dispergierung mit einem Plastikdecke abgedeckt. Zur Entfernung von eingeschlossenen Luftbläschen wird die Probe kurzzeitig evakuiert.

Nach einer Standzeit von 1 Stunde und 45 Minuten wird die Probe in den Meßbecher eingefüllt. Der Meßbecher wird in den Temperierkopf eingeschraubt und bei 22 °C temperiert. Nach 15 Minuten Standzeit erfolgt die Viskositätsmessung bei einem Schergefälle von $D = 2,72\,s^{-1}$. Der Zeiger am Anzeigeninstrument wird nach 30 Sekunden abgelesen. Nach dieser Zeit ist ein annähernd konstanter Wert erreicht.

4. Auswertung

Aus der vorgelegten Geschwindigkeit, dem Zeigerausschlag und der Eichkonstante des verwendeten Drehkörpers erhält man die Viskosität nach der Gleichung :

$\eta$ = U. S. K (mPas) U — Geschwindigkeitsstufe des Rotovisko (Bei RVI = 162)
S — angezeigte Skalenteile
K — Eichkonstante des verwendeten Drehkörpers.

Testrezeptur für Zahnpasten

Zusammensetzung der Phosphatgrundmasse :

| | |
|---|---|
| Wasser | 35,05 |
| Dehydazol A 400 P | 0,80 |
| Solbrol M-Na | 0,15 |
| Glyzerin | 12,00 |
| Sorbitol | 15,00 |
| Dicalziumphosphat-Dihydrat (L) | <u>34,00</u> |
| | 97,00 |

In 97 g dieses Vorkonzentrates wurden 3 g pyrogene Kieselsäure mit einem Spatel eingearbeitet und anschließend 3 x auf einem Dreiwalzenstuhl homogenisiert. Anschließend wurde die eingeschlossene Luft im Exsiccator entfernt. Von der so hergestellten Paste wurde nach 1. Tag die Viskosität mit dem Rotovisko System PK Nr. 8012 Geschwindigkeit 27 bestimmt.

**Anspruch**

Verfahren zur pyrogenen Herstellung von Kieselsäure mit einer spezifischen BET-Oberfläche von $250 \pm 25$ bis $350 \pm 25$ m$^2$/g und einer Verdickung von 4 000 bis 8 000 mPas in ungesättigten Polyestern, dadurch gekennzeichnet, daß man Siloxan oder ein Gemisch von verschiedenen Siloxanen in Gegenwart von Wasserstoff oder einem Kohlenwasserstoff verbrennt.

**Claim**

A process for the pyrogenic production of silica having a specific BET surface of from 250 to $\pm$ 25 to $350 \pm 25$ m$^2$/g and a thickening of from 4 000 to 8 000 mPas in unsaturated polyesters, characterised in that a siloxane or a mixture of various siloxanes is burnt in the presence of hydrogen or a hydrocarbon.

**Revendication**

Procédé pour la fabrication par pyrogénation d'acide silicique ayant une surface spécifique BET de $250 \pm 25$ à $350 \pm 25$ m$^2$/g et donnant un épaississement de 4 000 à 8 000 mPas dans des polyesters insaturés, procédé caractérisé en ce qu'on fait brûler un siloxane ou un mélange de différents siloxanes en présence d'hydrogène ou d'un hydrocarbure.